# EUROPEAN PATENT APPLICATION

(11) **EP 3 103 480 A1**
(43) Date of publication of application: **14.12.2016**
(21) Application number: 16180438.0
(22) Date of filing: 18.05.2012
(51) Int. Cl.: A61L 9/01

(54) **LOW VOLATILE REACTIVE MALODOR COUNTERACTIVES AND METHODS OF USE THEREOF**

(30) Priority: 20.05.2011 US 201113112622
(62) Divisional of application: 12168566.3
(71) Applicant: International Flavors & Fragrances Inc., New York, NY 10019 (US)
(72) Inventor: PLUYTER, Johan Gerwin Lodewijk, Middletown, NJ 07748 (US); HUANG, Xiao, Freehold, NJ 07728 (US); SASAKI, Takashi, Belford, NJ 07718 (US)
(74) Representative: Lawrence, John

(57) **Abstract**

The present invention is a compound for counteracting thiol- and amine-based malodors in consumer, industrial and textile products. The compound comprises an α,β-unsaturated carbonyl moiety covalently attached to an alkene oxide polymer or block or random copolymer thereof.

## Description

### Background of the Invention

Malodors are offensive odors, which are encountered in the air and on many substrates such as fabrics, hard surfaces, skin, and hair. Amines, thiols, sulfides, short chain aliphatic and olefinic acids, e.g. fatty acids, are typical of the chemicals found in and contributed to sweat, household, and environmental malodors. These types of malodors typically include indole, skatole, and methanethiol found in toilet and animal odors; piperidine and morpholine found in urine; pyridine and triethyl amine found in kitchen and garbage odors; and short chain fatty acids, such as 3-methyl-3-hydroxyhexanoic acid, 3-methylhexanoic acid or 3-methyl-2-hexenoic acid, found in axilla malodors. Compounds which have been found in the axilla are described for example by Xiao-Nong Zeng, et al. (1991) J. Chem. Ecol. 17:1469-1492.

Malodor counteractants or masking agents have been described in the art. For example, sulthydryl reactants, such as diethyl fumarate, di-n-butyl maleate and N-ethylmaleimide are disclosed in US 5,601,809 as compounds that are effective against axillary malodor. Further, the use of certain aromatic unsaturated carboxylic acid esters in combination with alkyl fumarates as malodor counteractants is disclosed in WO 2002/051788. US 6,403,075 addresses fragrance materials with a phenyl ring moiety as ammonia masking agents. Similarly, US 2002/0058017 describes cis-3-hexenol to mask ammonia. Moreover, US 7,585,833 describes methods for formulating fragrances to mask malodor present in products containing ammonia and substituted amines. See also US 6,379,658, US 6,376,741, US 5,769,832 and US 5,037,412.

Although the art describes compositions and methods for neutralizing certain malodors, there still remains a need for additional compounds that are more efficient against malodors.

### Summary of the Invention

The present invention features a malodor counteractant compound composed of a α,β-unsaturated carbonyl moiety covalently attached to a polymer, oligomer, surfactant or solid surface. In one embodiment, the α,β-unsaturated carbonyl moiety of the malodor counteractant compound is an ionone, an irone, a damascone, an acryloxy or methacryloxy group, or a crotonate. In other embodiments, the polymer is a polyol, polysaccharide, polyamine, polyacrylate, alkene oxide polymer, or block or random copolymer thereof; the oligomer is an oligosaccharide or oligomeric alkane; the surfactant is a poloxamine or an unbranched C₁₃C₁₅ oxo alcohol; and the solid surface is a silica or clay surface. In still other embodiments, the malodor counteractant has the structure: 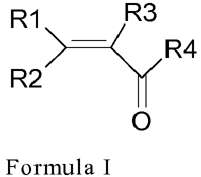 wherein, at least one of R1, R2, R3 or R4 is a polymer, oligomer, solid surface, or surfactant; and at least one of R1, R2, R3 or R4 is selected from the following group of H, a saturated aliphatic group, an unsaturated aliphatic group, an aromatic group, a heterocyclic group, O-R5, NH-R5, an polyalkylene oxide group or a blocked structure thereof, a nonionic fatty alcohol group, an ester of a nonionic fatty alcohol group, an amide of a nonionic fatty amine group or an ethoxylated fatty amine group. Consumer, industrial and textile products containing the malodor counteractant are also provided as are methods for producing the malodor counteractant and using the malodor counteractant to counteract a thiol- or amine-based malodor.

### Detailed Description of the Invention

The present invention provides compounds composed of an α,β-unsaturated carbonyl moiety covalently attached to a polymer, surfactant (nonionic with OH, NH₂ or COOH groups) or solid surface (silica, clay) for use as malodor counteractants. Advantageously, the α,β-unsaturated carbonyl moiety of the instant malodor counteractant compounds binds to thiol- and amine-based malodors thereby effectively reducing the concentration of these malodors in consumer, industrial or textile products. Moreover, the instant compounds have a low vapor pressure such that the compounds can be added in significant quantities to products without impacting the olfactory character of the products. Given these features, the instant compounds find use as additives to consumer products to reduce the concentration of malodors in the headspace of the product. Furthermore, the instant compounds can be used to form a fragrance or flavor encapsulate or other delivery system such that while the delivery system is delivering its payload, malodors are removed from the air. Alternatively, the instant compounds can be formulated into a product, such as a fragrance, which can be optionally formulated into a delivery system.

As indicated, particular embodiments feature compounds with low-to-no vapor pressure. Vapor pressure (P°) is the pressure of a vapor of a compound in equilibrium with its pure condensed phase (solid or liquid). Vapor pressure is measured in the standard units of pressure. The International System of Units (SI) recognizes pressure as a derived unit with the dimension of force per area and designates the pascal (Pa) as its standard unit. One pascal is one Newton per square meter (N·^{m-2} or kg·^{m-1}·^{s-2}). Vapor pressures depend on the temperature and vary with different compounds due to differences in molecule-molecule interactions. For example, vapor pressure at 25°C of n-alkanes is a function of chain length, wherein larger n-alkane molecules have lower P° due to greater polarizability and increased strength of London Dispersion intermolecular forces. The vapor pressure of a compound can be determined by conventional methods known to those of skill in the art. In particular embodiments, compounds of the invention have a vapor pressure of less than 200 Pa (1.5 mmHg), less than 100 Pa (0.75 mmHg), less than 50 Pa (0.375 mmHg), less than 20 Pa (0.15 mmHg), or less than 10 Pa (0.075 mmHg), at 25°C.

The α,β-unsaturated carbonyl moiety of the instant malodor counteractant compound is, in particular embodiments, an α,β-unsaturated ketone, ester or amide species. Accordingly, in certain embodiments, the instant compound has the structure: 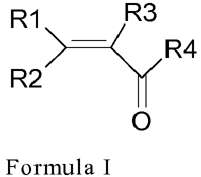 wherein at least one of R1 to R4 is a polymer, oligomer, solid surface, surfactant or other low volatile organic or inorganic molecule; and at least one of R1 to R4 is selected from the following group of H, a saturated aliphatic group, an unsaturated aliphatic group, an aromatic group, a heterocyclic group, O-R5 (an ester for R4), NH-R5 (an amide for R4), an polyalkylene oxide group or a blocked structure thereof, a nonionic fatty alcohol group, an ester of a nonionic fatty alcohol group, an amide of a nonionic fatty amine group or an ethoxylated fatty amine group, wherein said group can contain a negatively charged or positively charged functional group including, but not limited to, an amine, sulfate, sulfonate, carboxylic acid or phenolic functional group. In some embodiments, the atom connecting the keto and the R4 group can be either C, O or N. In particular embodiments, the R4 group is linked by C or N so that the hydrolysis stability of the compound is increased.

In embodiments where at least one of R1 to R4 is O-R5 or N-R5, R5 is desirably selected from the group of a saturated aliphatic group, an unsaturated aliphatic group, an aromatic group, a heterocyclic group, an polyalkylene oxide group or a blocked structure thereof, a nonionic fatty alcohol group, an ester of a nonionic fatty alcohol group, an amide of a nonionic fatty amine group or an ethoxylated fatty amine group, a polymer, an oligomer, a solid surface, a surfactant or a low volatile organic or inorganic compound.

As is conventional in the art, an "aliphatic group" is an organic molecule whose carbon atoms are linked in open chains, either straight or branched. Examples of aliphatic groups include, but are not limited to, alkanes, alkenes, and alkynes.

"Aromatic group" means a monovalent group having a monocyclic ring structure or fused bicyclic ring structure. Monocyclic aromatic groups contain 5 to 10 carbon atoms, preferably 5 to 7 carbon atoms, and more preferably 5 to 6 carbon atoms in the ring. Bicyclic aromatic groups contain 8 to 12 carbon atoms, preferably 9 or 10 carbon atoms in the ring. Aromatic groups are unsubstituted. The most preferred aromatic group is phenyl.

"Heterocyclic group" means a saturated or unsaturated ring structure containing carbon and 1 to 4 heteroatoms in the ring. Heterocyclic groups are monocyclic, or are fused or bridged bicyclic ring systems. Monocyclic heterocyclic groups contain 4 to 10 member atoms (*i*.*e*., including both carbon atoms and at least 1 heteroatom), preferably 4 to 7, and more preferably 5 to 6 in the ring. Bicyclic heterocyclic groups contain 8 to 12 member atoms, preferably 9 or 10 in the ring. Preferred heterocyclic groups include piperzyl, morpholinyl, tetrahydrofuranyl, tetrahydropyranyl, and piperdyl.

A polyalkylene oxide group of the invention is an alkyl phenol having from 4 to 25, preferably 4-16, moles of ethylene oxide per mole of alkyl phenol. Examples of polyalkylene oxide groups include, but are not limited to polyethylene glycol and polyethylene oxide.

Fatty alcohols are aliphatic alcohols composed of a chain of 8 to 22 carbon atoms. Fatty alcohols typically have an even number of carbon atoms and a single alcohol group (-OH) attached to the terminal carbon. Fatty alcohols can be unsaturated or branched. Due to their amphipathic nature, fatty alcohols behave as nonionic surfactants.

A polymer in accordance with the instant invention is a molecule composed of repeating monomer units. In contrast to a polymer, which can contain numerous monomers, an oligomer is a molecule that is composed of a few monomer units. In this respect, oligomers include dimers, trimers, tetramers, and the like. According to the present invention, a polymer includes, but is not limited to, a polyol (*e.g.,* poly vinyl alcohol and its copolymers), polysaccharide (*e.g*., maltodextrin), polyamine (*e.g.,* poly vinyl amines and its copolymers), polyacrylate with alcohol groups, alkylene oxide polymer with OH or NH₂ end groups (*e.g*., polyethylene oxide (PEG)) and block and random copolymer forms thereof. Oligomers include, but are not limited to, *e.g.,* oligosaccharides and oligomeric alkanes (*e.g.,* pentanes, butanes, or hexane).

In one embodiment, solid surfaces of the invention include, but are not limited to a silica surface (*e.g*., a synthetic amorphous silica surface such as SYLOID), clay or other solid mineral materials with an appropriate functional group to attaching the α,β-unsaturated carbonyl moiety. In another embodiment, the solid surface is the surface of a delivery system such as a nanoparticle, microparticle, nanocapsule, or microcapsule, containing one or more α,β unsaturated carbonyl moieties as described herein.

Surfactants are compounds that lower the surface tension of a liquid, the interfacial tension between two liquids, or that between a liquid and a solid. Surfactants may act as detergents, wetting agents, emulsifiers, foaming agents, and dispersants. Surfactants are usually organic compounds that are amphiphilic. Surfactants include molecules such as PLURONIC surfactants (based on ethylene oxide, propylene oxide and/or butylenes oxide as di- and tri-block copolymers) and TETRONIC surfactants (poloxamine or block copolymers based on ethylene oxide and propylene oxide with a vapor pressure of <0.1 mmHg at 25°C) including TETRONIC 901, TETRONIC 701, TETRONIC 90R4, and TETRONIC 904, and LUTENSOL AO nonionic surfactants (unbranched C₁₃C₁₅ oxo alcohol) including LUTENSOL AO3, LUTENSOL AO4, LUTENSOL AO5, and LUTENSOL AO7.

According to particular embodiments, the α,β-unsaturated carbonyl moiety is an odorant or fragrance such as an ionone, irone, damascone or a structurally related molecule. Examples of this class of molecules include α-damascone, β-damascone, δ-damascone, iso-damascone, γ-methylionone, dynascone, α-irone, dihydroirone, α-ionone, β-ionone and trimethylcyclohexyl)-pent-1-en-3-one. In another embodiment, the α,β-unsaturated carbonyl moiety is a crotonate such as geranyl crotonate, ethyl crotonate, methyl crotonate, benzyl crotonate and the like. In yet another embodiment, the α,β-unsaturated carbonyl moiety is an acryloxy or methacryloxy group. Examples of such a group include, but are not limited to, glycidyl methacrylate. Moreover, the acryloxy group can incorporated into the instant compound via the carboxyl group of an acrylate monomer. In this respect, compounds of the present invention can be prepared by acrylation of a polymer, an oligomer, a solid surface, a surfactant or a low volatile organic or inorganic compound. In particular embodiments, the α,β-unsaturated carbonyl moiety is *e.g.,* acetic acid 2,5-dimethyl-bicyclo[3.2.1]oct-2-en-3-yl ester; acetic acid 1,4-dimethyl-bicyclo[3.2.1]oct-2-en-3-yl ester; 4-allyl-1,4-dimethyl-bicyclo[3.2.1]octan-3-one or a derivative thereof.

In particular embodiments, the malodor counteractant compound of the present invention is a small molecule with no-to-low vapor pressure attached to one or more α,β-unsaturated carbonyl groups. In another embodiment, the malodor counteractant compound of the present invention is a polymer material attached to one or more α,β-unsaturated carbonyl groups. In a further embodiment, the malodor counteractant compound of the present invention is a small molecule or oligomer with a surfactant character, with one or more α,β-unsaturated carbonyl groups attached thereto. In still a further embodiment, the malodor counteractant compound of the present invention is a insoluble solid attached to one or more α,β-unsaturated carbonyl groups.

Specific examples of malodor counteractant compounds of the invention include, but are not limited to, the compounds provided in the Examples and in Table 1.

**TABLE 1**

| | |
|---|---|
| 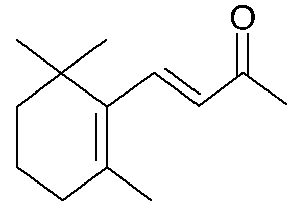 | 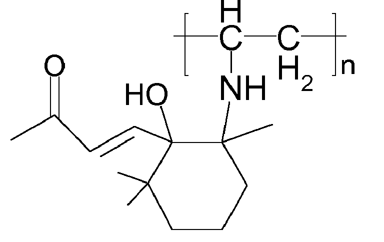 |
| 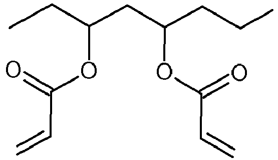 | 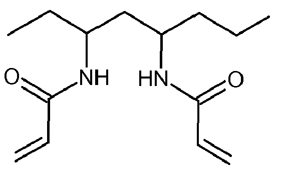 |
| 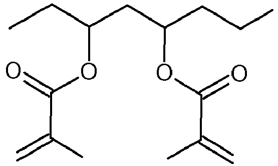 | 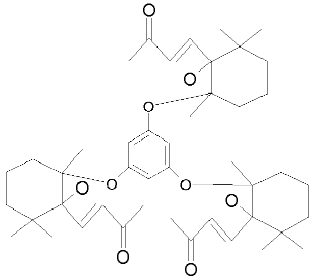 |
| 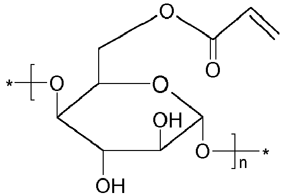 | 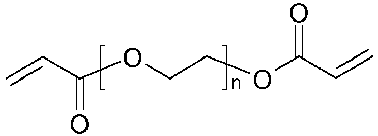 |
| 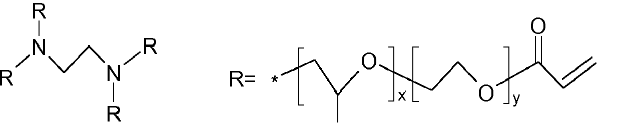 | |
| 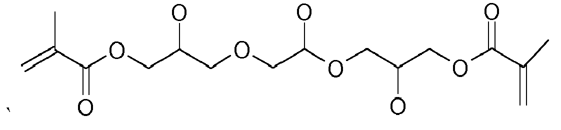 | |
| 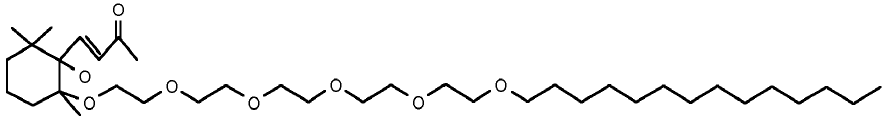 | |
| 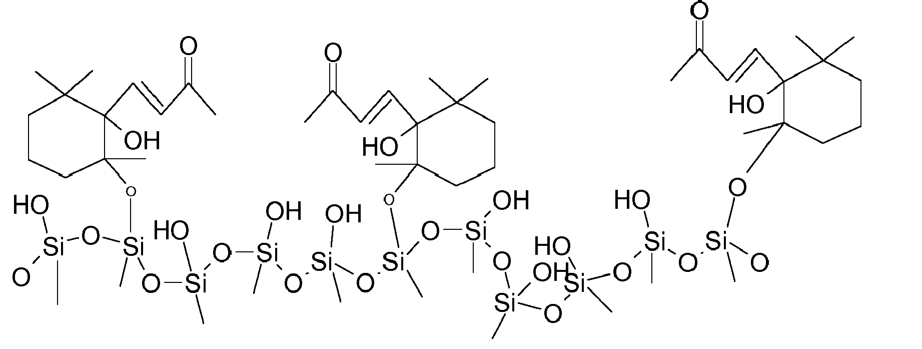 | |
| 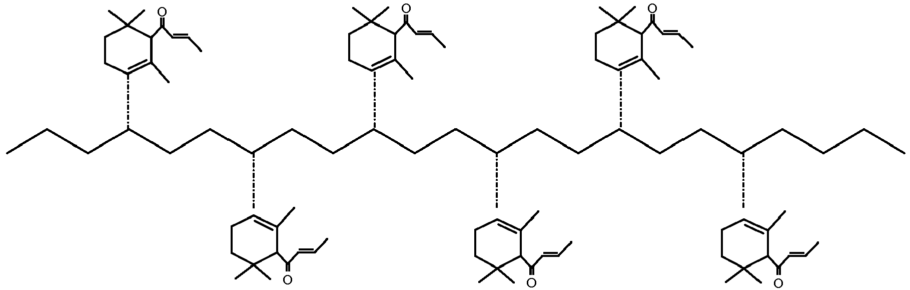 | |

Malodor counteractant compounds of the invention are produced by covalently attaching a α,β-unsaturated carbonyl moiety as described herein to a polymer, oligomer, surfactant or solid surface. Given that the α,β-unsaturated carbonyl moiety is covalently attached, this moiety is not released before or during use in a consumer, industrial or textile product, *e*.*g*., the instant compound is not a pro-fragrances. Specific examples of reagents and reactions conditions for preparing the instant compounds are provided in Example 1.

The instant malodor counteractant compounds can be used in a variety of forms and in a variety of products. Advantageously, the instant compounds are reactive against potent malodor ingredients while not affecting the odor of a fragrance or final product. Furthermore, these compounds and the methods herein can be pursued in any situation where malodor is present. In this respect, the present invention also features a method for counteracting amine- and thiol-based malodors of consumer, industrial and textile products, as well as the surrounding environment, by introducing or adding one or more malodor counteractant compounds of the invention to a consumer, industrial or textile product so that thiol- or amine-based malodors of the product are counteracted. For the purposes of the present invention, a compound counteracts a malodor if it measurably (either qualitatively or quantitatively) reduces the presence of a malodor. In particular embodiments, a compound of the invention reduces the presence of an amine- and thiol-based malodor of a product by 50-100% as compared to a product that does not have the malodor counteractant compound.

In embodiments where the instant compound is intended to target a thiol-based malodor, it is particular advantageous to add an amine or other base including Lewis acids and Brønsted acids to accelerate the reactivity of the thiols with the α,β-unsaturated carbonyl compound of the invention. An example of a suitable Brønsted acid is bis(trifluoromethanesulfon)imide, which has been shown to catalyze the addition of thiols to α,β-unsaturated ketones, alkylidene malonates and acrylimides (Wabnitz & Spencer (2003) Organic Lett. 5:2141-2144). In some embodiments, the amine is already a component of the consumer, industrial or textile product. In other embodiments, the amine is an additional component added with the α,β-unsaturated carbonyl compound of the invention. Amines of use in the instant invention include, but are not limited to primary aliphatic amines, secondary aliphatic amines, tertiary aliphatic amines, aromatic amines, and heterocyclic amines. As is conventional in the art, a primary aliphatic amine is composed of one alkyl substituent bound to N together with two hydrogens. Examples of primary aliphatic amines include methylamine, ethanolamine (2-aminoethanol), and the buffering agent tris. Secondary amines have two alkyl substituents bound to nitrogen together with one hydrogen. Examples of secondary aliphatic amines include dimethylamine and methylethanolamine. In tertiary amines, all three hydrogen atoms are replaced by organic substituents. Examples include trimethylamine. An aromatic amine is an amine with an aromatic substituent (*i*.*e*., -NH₂, -NH- or nitrogen group(s) attached to an aromatic hydrocarbon) whose structure usually contains one or more benzene rings. Examples of aromatic amines include, but are not limited to, aniline, toluidine, 2,4,6-trimenthylaniline, and anisidine. A heterocyclic amine is a compound containing at least one heterocyclic ring, which by definition has atoms of at least two different elements, wherein at least one of the atoms of the ring is a nitrogen. Examples of heterocyclic amines include, but are not limited to, indoles, quinolines, quinoxalines, and pyridines.

Malodors particularly targeted by the instant molecules include amine- and thiol-based malodor such as bathroom odors, sweat, food odors, textile odors, home care and personal care product base odors, adhesive odors, and paint odors. In this respect, the instant compounds can be used in air refresheners, fabric refresheners, bar soaps, perfumes, fragrances, cologne, bath or shower gels, shampoos or other hair care products, cosmetic preparations, body odorants, deodorants, antiperspirants, liquid or solid fabric detergents or softeners, bleach products, disinfectants or all-purpose household or industrial cleaners, food, or industrial or textile products such as adhesives, paints, coatings, or textiles. In yet another embodiment, one or more of the instant compounds are used as part of a delivery system or polymer system to deliver a fragrance or compound of interest (*e.g*., a pharmaceutical).

Aspects of the invention may also be provided by the following numbered paragraphs.
1. A malodor counteractant compound comprising a α,β-unsaturated carbonyl moiety covalently attached to a polymer, oligomer, surfactant or solid surface.
2. A consumer, industrial or textile product comprising a malodor counteractant having a α,β-unsaturated carbonyl moiety covalently attached to a polymer, oligomer, surfactant or solid surface.
3. A method for producing a malodor counteractant comprising covalently attaching a α,β-unsaturated carbonyl moiety to a polymer, oligomer, surfactant or solid surface.
4. The malodor counteractant compound of paragraph 1, or the consumer, industrial or textile product of paragraph 2, or the method of paragraph 3, wherein the α,β-unsaturated carbonyl moiety comprises an ionone, an irone, a damascone, an acryloxy group, a methacryloxy group or a crotonate.
5. The malodor counteractant compound of paragraph 1 or paragraph 4, or the consumer, industrial or textile product of paragraph 2, or of paragraph 4, or the method of paragraph 3 or of paragraph 4, wherein the polymer comprises a polyol, polysaccharide, polyamine, polyacrylate, alkene oxide polymer, or block or random copolymer thereof.
6. The malodor counteractant compound of paragraph 1 or of paragraph 4 or of paragraph 5, or the consumer, industrial or textile product of paragraph 2 or of paragraph 4 or of paragraph 5, or the method of any one of paragraphs 3 to 5, wherein the oligomer comprises an oligosaccharide or oligomeric alkane.
7. The malodor counteractant compound of paragraph 1 or of any of paragraphs 4 to 6, or the consumer, industrial or textile product of any of paragraphs 4 to 6, or the method of paragraph 3 or of any of paragraphs 4 to 6, wherein the surfactant comprises a poloxamine or an unbranched C₁₃C₁₅ oxo alcohol.
8. The malodor counteractant compound of paragraph 1 or of any of paragraphs 4 to 7, or the consumer, industrial or textile product of any preceding paragraph, or the method of any preceding paragraph, wherein the solid surface comprises a silica or clay surface.
9. The malodor counteractant compound of paragraph 1 or of any of paragraphs 4 to 7, or the consumer, industrial or textile product of paragraph 2 or of any of paragraphs 4 to 7, or the method of paragraph 3 or of any of paragraphs 4 to 7, wherein the solid surface comprises the surface of a delivery system containing one or more α,β-unsaturated carbonyl moieties.
10. The malodor counteractant compound of paragraph 1, wherein the malodor counteractant has the structure: 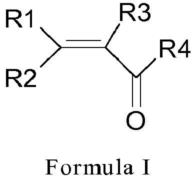 wherein, at least one of R1, R2, R3 or R4 is a polymer, oligomer, solid surface, or surfactant; and at least one of R1, R2, R3 or R4 is selected from the following group of H, a saturated aliphatic group, an unsaturated aliphatic group, an aromatic group, a heterocyclic group, O-R5, NH-R5, an polyalkylene oxide group or a blocked structure thereof, a nonionic fatty alcohol group, an ester of a nonionic fatty alcohol group, an amide of a nonionic fatty amine group or an ethoxylated fatty amine group; and R5 is R5 is selected from the group of a saturated aliphatic group, an unsaturated aliphatic group, an aromatic group, a heterocyclic group, an polyalkylene oxide group or a blocked structure thereof, a nonionic fatty alcohol group, an ester of a nonionic fatty alcohol group, an amide of a nonionic fatty amine group, an ethoxylated fatty amine group, a polymer, an oligomer, a solid surface, or a surfactant.
11. The consumer, industrial or textile product of paragraph 2, or of any preceding paragraph, wherein the malodor counteractant has the structure: 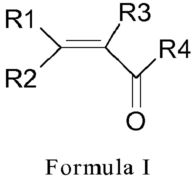 wherein, at least one of R1, R2, R3 or R4 is a polymer, oligomer, solid surface, or surfactant; and at least one of R1, R2, R3 or R4 is selected from the following group of H, a saturated aliphatic group, an unsaturated aliphatic group, an aromatic group, a heterocyclic group, O-R5, NH-R5, an polyalkylene oxide group or a blocked structure thereof, a nonionic fatty alcohol group, an ester of a nonionic fatty alcohol group, an amide of a nonionic fatty amine group or an ethoxylated fatty amine group; and R5 is R5 is selected from the group of a saturated aliphatic group, an unsaturated aliphatic group, an aromatic group, a heterocyclic group, an polyalkylene oxide group or a blocked structure thereof, a nonionic fatty alcohol group, an ester of a nonionic fatty alcohol group, an amide of a nonionic fatty amine group, an ethoxylated fatty amine group, a polymer, an oligomer, a solid surface, or a surfactant.
12. A method for counteracting a thiol- or amine-based malodor comprising adding the malodor counteractant compound of paragraph 1, or of any of paragraphs 4 to 10, to a consumer, industrial or textile product so that thiol- or amine-based malodors of the product are counteracted.
13. The method of paragraph 12, further comprising adding an amine, Lewis acid or Brønsted acid to the consumer, industrial or textile product.
14. The method of paragraph 13, wherein the amine comprises a primary aliphatic amine, secondary aliphatic amine, tertiary aliphatic amine, aromatic amine, or heterocyclic amine.

The invention is described in greater detail by the following non-limiting examples.

### Example 1 Synthetic Protocols

*SYLOID 244 Modified with Beta-Ionone Epoxide.* Beta ionone epoxide (4g) and imidazole (1.25g) were dissolved in 50g toluene in a round-bottom flask. SYLOID 244 (5 g; W.R. Grace & Co.) was added to the flask and the mixture was refluxed at 110°C for ~2 hours. The sample was vacuum filtered to collect the solid, which was subsequently washed several times with toluene. The results solid was dried in a fume hood overnight.

*LUPAMIN 9095 Modified with Beta-Ionone Epoxide.* Beta ionone epoxide (4g) was dissolved in 30g methanol in a round-bottom flask. LUPAMIN 9095 (20 g; BASF) was slowly added to the flask with stirring under nitrogen gas. The mixture was incubated at ~58°C for 2 days. The mixture was precipitated in large excess of acetone/tetrahydrofuran (THF) (2:1 v/v). The precipitate was vacuum filtered to collect the solids and the solids were subsequently washed twice with acetone/THF (2:1 v/v), each followed by vacuum filtration. The solids were dried in an oven at ~70°C for at least 24 hours.

*Maltodextrin Modified with Beta-Ionone Epoxide.* Maltrin QD M585 (16.2g; Grain Processing Corp.) and beta-ionone epoxide (20.8g) were dissolved in 70g dimethyl sulfoxide (DMSO) in a round-bottom flask at 62°C under nitrogen gas. *N,N,N',N'-*tetraethylmethylenediamine (TEMED; 151 µl) was added to the flask and the reaction was incubated at 62°C for 6 hours. Subsequently the reaction mixture was cooled to room temperature and was precipitated in a large excess of methanol/acetone (1:9 v/v). The solids were collected by vacuum filter and washed twice with methanol/acetone (1:9 v/v), each wash followed by vacuum filtration. The product was dried under vacuum at ~50°C for at least 24 hours.

*LUTENSOL AO7 Modified with Beta-Ionone Epoxide.* LUTENSOL AO7 (26 g; BASF) and beta-ionone epoxide (20.8 g) were combined in a round-bottom flask and heated to ~65°C under nitrogen gas until a clear solution was obtained. TEMED (75 µl) was added to the flask and the reaction was allowed to proceed at ~65°C for 6 hours. DI H₂O (46 g) and hexanes (46 g) were added, and the mixture was allowed to settle at 60°C overnight. The next day, while the mixture was warm, the upper clear liquid layer (bottom layer was a white emulsion/precipitation) was removed and placed in a beaker to cool to room temperature. Once cooled, the clear liquid layer turned into an elastic soft gel-like material. This gel-like material was washed 2 to 3 times with hexanes, which were decanted after each wash. The washed gel-like material was dried in a vacuum at ~40°C for at least 24 hours. Once dried, the material again turned into a clear viscous liquid product.

*Silica Surface Modified with Beta-Ionone Epoxide.* β-Ionone was modified with an epoxy group using conventional methods. The resulting epoxy-modified β-ionone was combined with a silica surface so that the β-ionone was covalently attached by reaction of the silanol group with the epoxy group.

*Methacrylation of Maltodextrin*. Maltrin QD M585 (16.2 g) was dissolved in 40 g DMSO in a round-bottom flask at ~62°C under nitrogen gas. Glycidyl methacrylate (14.2 g) was added slowly and TEMED (151 µl) was subsequently added very slowly. The reaction was allowed to proceed at ~62°C for 6 hours. The reaction mixture was subsequently precipitated in a large excess of methanol/acetone (3:7 v/v) and vacuum filtered to collect solids. The solids were washed twice with methanol/acetone (3:7 v/v), wherein each wash was followed by vacuum filtration. The solids were then dried under vacuum at ~50°C for at least 24 hours.

*Acrylation of Maltodextrin.* Maltrin QD M585 (16.2 g) was dissolved in 60 g dimethylformamide (DMF) in a round-bottom flask. Triethylamine (TEA; 20.2 g) was added and the mixture was cooled to ∼0°C in an ice bath under nitrogen gas. Acryloyl chloride (18.1 g) was mixed with 20 g DMF, and this mixture was added drop-wise to the flask while stirring in ice bath. The reaction was allowed to proceed at ∼0°C for 3 hours and then at room temperature for -18 hours. Solid triethylammonium chloride by-product was filter out and the clear yellowish filtrate was collected. The filtrate was precipitated in a large excess of THF/2-propanol (IPA) (8:2 v/v) and was vacuum filtered to collect the solids. The solids were re-dissolved in ~50 g DMSO and subsequently precipitated in a large excess of THF/IPA (1:9 v/v). The solids were collected by vacuum filter and washed twice with THF/IPA (1:9 v/v), wherein each wash was followed by vacuum filtration. The resulting solids were dried under vacuum at ~50°C for at least 24 hours.

### Michael Addition of a-Damascone with Pentane Thiol (Scheme 1).

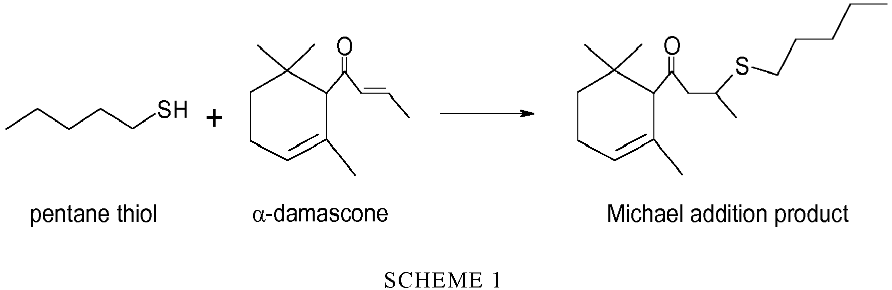

### Addition of Pentane Thiol onto Poly Vinyl Amine via an Epoxy Functional Group (Scheme 2).

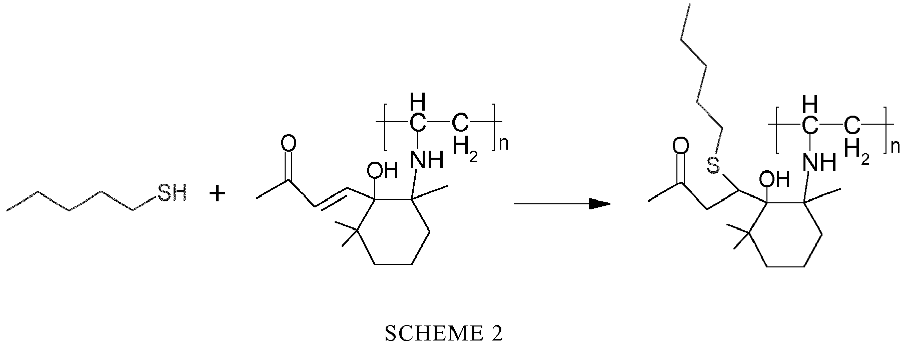

### Addition of □, □□ - Unsaturated Carbonyl Moiety to Polymers (Scheme 3).

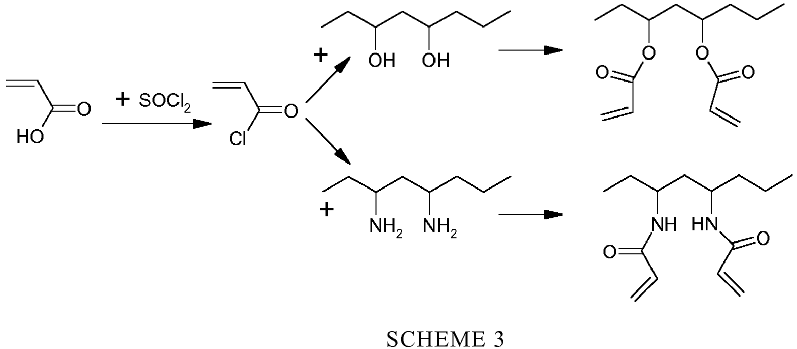

### Addition of Glycidyl Methacrylate to Poly Vinyl Alcohol (Scheme 4).

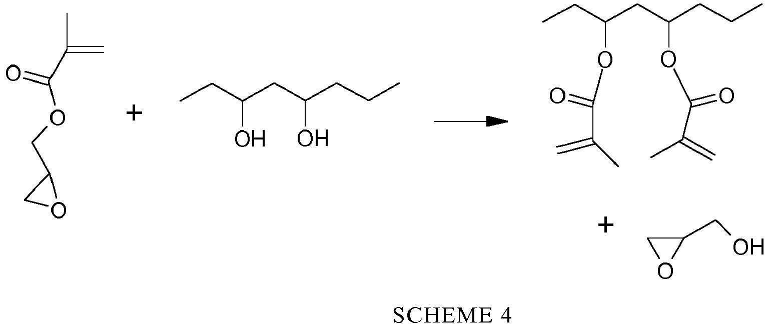

### Example 2: Testing for Malodor Counteractive Reactivity

This method was applicable to water-soluble, modified polymers/surfactants. In general, the reactivity of a compound was measured by adding a dilute solution of n-Propanethiol (n-Prt) to an aqueous solution of the compound to be tested. After a set period of time, an aliquot of headspace above the reaction solution was sampled and injected into the gas chromatograph for separation and detection. The concentration of compound used was adjusted so that the final molar ratio of the compound and n-Prt was about 2:1. For polymers, an averaged molecular weight was used for this calculation.

More specifically, aqueous malodor (n-Prt) solution was obtained by preparing a 1% (wt/wt) solution of n-Prt in MeOH. The 1% solution was then diluted to 0.05% (wt/wt), which was equivalent to 500 ppm. Both solutions were stored at in a 0°C refrigerator when not in use.

Aqueous reactive polymer/surfactant solutions (50 to 100 mL) were prepared with distilled (DI) water and thoroughly mixed with a magnetic stir bar. The amount of reactive polymer/surfactant used was determined by calculating the concentration of polymer needed to provide a 2:1 ratio with n-Prt. Using the same calculations, solutions of "unmodified" polymer/surfactant were prepared for comparison.

For analysis, 1 mL of aqueous polymer/surfactant was placed into a 20 mL headspace vial using a positive displacement pipette. To the aqueous polymer/surfactant in the vial was added 250 µL of 0.05% n-Prt solution. After addition, the vial was immediately capped. After all samples were capped, the vials were placed in a holder and set on an orbital incubator for a preselected mixing/equilibration time.

The results of this analysis for selected compounds are presented in Table 2.

**TABLE 2**

| Malodor Counter-actant | Properties | Performance vs Water Alone | Performance vs Original Polymer |
|---|---|---|---|
| Acrylated Tetronic 904 | Jelly-like, soluble in water, Mn~6700 | 56% (2.2% in water), 72 hours equilibration | 61% (2.2% in water), 72 hours equilibration |
| Acrylated Tetronic 90R4 | Jelly-like, soluble in water, Mn~6900 | 66% (2.3% in water), 72 hours equilibration | 0% (2.3% in water), 72 hours equilibration |
| Acrylated Tetronic 901 | Viscous liquid, Insoluble in water, Mn~4700 | N/A | N/A |
| Acrylated maltodextrin | Solid, Soluble in water | 24% (0.8% in water), 1 hour equilibration | 8% (0.8% in water), 1 hour equilibration |
| Acrylated PEG | Solid, Soluble in water, Mn~3400 | 99% (1.1% in water), 72 hour equilibration | 99% (1.1% in water), 72 hour equilibration |

| | | | |
|---|---|---|---|
| Stoichiometry (mole-based) used: 2:1, reactive groups of polymers:propane thiol | | | |

### Example 3: Reduction of Thiol Malodor by Malodor Counteractants

In additional analysis, the ability of select counteractive compounds to reduce the amount of thiol malodor was determined. The results of this analysis are presented in Table 3.

**TABLE 3**

| Malodor Counter-actant | % "Thiol" Reduction | Solvent |
|---|---|---|
| Maltodextrin-glycidyl methacrylate 25/75 (at 1%) | 25-50% | Water |
| Ethylene glycol methyl ether acrylate | 98% | 20% n-propanol in water |
| Methyl crotonate @ T₀ & T_{12 hr} (monomer) | 88/100% | neat/DEP |
| Ethyl crotonate @ T₀ & T_{12 hr} (monomer) - | 43/95% | neat/DEP |

### Example 4: Acceleration of Thiol Reactivity Using Amines

It was also found that the reactivity of thiols with □□□-unsaturated carbonyl compounds can be accelerated by the presence of amines such as primary, secondary and tertiary aliphatic and aromatic amines, heterocyclic amines, amines present in the malodor mixture, as well as basic ingredients such as hydroxides. An example of the extent of reactivity is shown in Scheme 5, with structures confirmed by NMR.

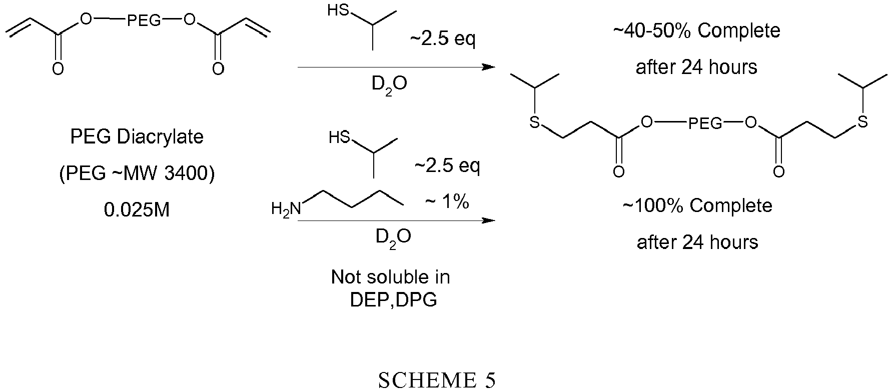

The catalysis of the same reaction with an indole was also carried out and is depicted in Scheme 6.

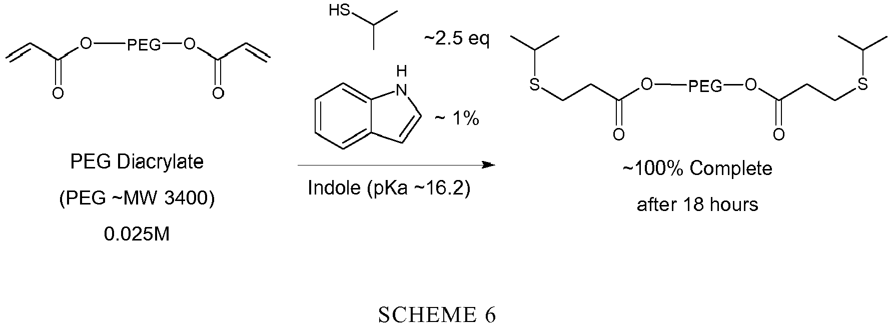

It was confirmed that the indole by itself did not have any reactivity with 2-propanethiol.

## Claims

1. A malodor counteractant compound comprising a α,β-unsaturated carbonyl moiety covalently attached to a polymer, wherein the polymer is an alkene oxide polymer or block or random copolymer thereof.

2. The malodor counteractant compound of claim 1, wherein the malodor counteractant compound has a vapor pressure of less than 200Pa.

3. The malodor counteractant compound of claim 1, wherein the malodor counteractant compound has a vapor pressure of less than 20Pa.

4. A consumer, industrial or textile product comprising a malodor counteractant having a α,β-unsaturated carbonyl moiety covalently attached to a polymer; wherein the polymer is an alkene oxide polymer or block or random copolymer thereof.

5. A method for producing a malodor counteractant comprising covalently attaching a α,β-unsaturated carbonyl moiety to a polymer; wherein the polymer is an alkene oxide polymer or block or random copolymer thereof.

6. The malodor counteractant compound of any one of claims 1-3, or the consumer, industrial or textile product of claim 4, or the method of claim 5, wherein the α,β-unsaturated carbonyl moiety comprises an ionone, an irone, a damascone, an acryloxy group, a methacryloxy group or a crotonate.

7. The malodor counteractant compound of any one of claims 1-3, wherein the malodor counteractant has the structure: 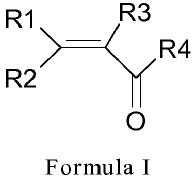 wherein, at least one of R1, R2, R3 or R4 is O-R5, NH-R5, an polyalkylene oxide group or a blocked structure thereof; and at least one of R1, R2, R3 or R4 is selected from the following group of H, a saturated aliphatic group, an unsaturated aliphatic group, an aromatic group, a heterocyclic group, a nonionic fatty alcohol group, an ester of a nonionic fatty alcohol group, an amide of a nonionic fatty amine group or an ethoxylated fatty amine group; and R5 is selected from the group of a saturated aliphatic group, an unsaturated aliphatic group, an aromatic group, a heterocyclic group, an polyalkylene oxide group or a blocked structure thereof, a nonionic fatty alcohol group, an ester of a nonionic fatty alcohol group, an amide of a nonionic fatty amine group, an ethoxylated fatty amine group, a polymer, an oligomer, a solid surface, or a surfactant.

8. The consumer, industrial or textile product of claim 4, or of any preceding claim, wherein the malodor counteractant has the structure: 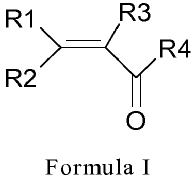 wherein, at least one of R1, R2, R3 or R4 is O-R5, NH-R5, an polyalkylene oxide group or a blocked structure thereof; and at least one of R1, R2, R3 or R4 is selected from the following group of H, a saturated aliphatic group, an unsaturated aliphatic group, an aromatic group, a heterocyclic group, a nonionic fatty alcohol group, an ester of a nonionic fatty alcohol group, an amide of a nonionic fatty amine group or an ethoxylated fatty amine group; and R5 is selected from the group of a saturated aliphatic group, an unsaturated aliphatic group, an aromatic group, a heterocyclic group, an polyalkylene oxide group or a blocked structure thereof, a nonionic fatty alcohol group, an ester of a nonionic fatty alcohol group, an amide of a nonionic fatty amine group, an ethoxylated fatty amine group, a polymer, an oligomer, a solid surface, or a surfactant.

9. A method for counteracting a thiol- or amine-based malodor comprising adding the malodor counteractant compound of any one of claims 1-3, or of any of claims 6 and 7, to a consumer, industrial or textile product so that thiol- or amine-based malodors of the product are counteracted.

10. The method of claim 9, further comprising adding an amine, Lewis acid or Brønsted acid to the consumer, industrial or textile product.

11. The method of claim 9, wherein the amine comprises a primary aliphatic amine, secondary aliphatic amine, tertiary aliphatic amine, aromatic amine, or heterocyclic amine.
